# EUROPÄISCHE PATENTSCHRIFT

(11) **EP 1 880 671 B1**
(45) Veröffentlichungstag und Bekanntmachung des Hinweises auf die Patenterteilung: **08.09.2010**
(21) Anmeldenummer: 06014898.8
(22) Anmeldetag: 18.07.2006
(51) Int. Cl.: A61B 5/155, A61B 5/00

(54) **Lanzettenrad**
Lancet wheel
Roue de lancettes

(43) Veröffentlichungstag der Anmeldung: 23.01.2008
(73) Patentinhaber: Roche Diagnostics GmbH, 68298 Mannheim (DE); F.HOFFMANN-LA ROCHE AG, 4070 Basel (CH)
(72) Erfinder: Hoenes, Joachim, 64673 Zwingenberg (DE); Zimmer, Volker, 67229 Laumersheim (DE); Voelkel, Dirk, 69469 Weinheim (DE); Haar, Hans-Peter, 69168 Wiesloch (DE); Quarder, Ortrud, 69115 Heidelberg (DE); Kuhr, Hans-Jürgen, 68219 Mannheim (DE)

(56) Entgegenhaltungen:
- DE-A1- 10 245 721
- DE-C1- 10 208 575
- US-A1- 2004 039 303

## Beschreibung

### Technisches Gebiet

Die Erfindung liegt auf dem Gebiet der Stechhilfen zur diagnostischen Ermittelung von Blutparametern.

Die Gewinnung und Analyse von Körperflüssigkeiten findet auf vielen Gebieten der medizinischen Diagnostik statt. Deshalb ist es wünschenswert auch Routinetests außerhalb des Laboratoriums schnell und reproduzierbar zu ermöglichen. Das Testen kann mit verschiedenen Körperflüssigkeiten durchgeführt werden, wie z. B. Blut und/oder interstitieller Flüssigkeit. Diese Flüssigkeiten können auf verschiedene Charakteristiken hin untersucht werden. Die Ergebnisse dieser Untersuchung sind wichtig, um verlässliche Diagnosen, therapeutische Maßnahmen und Therapieverfolgungen durchführen zu können.

Die eigenhändige Blutzuckerbestimmung (das sogenannte "Home Monitoring") ist heute eine weltweit verbreitete Methode in der Diabetes-Kontrolle. Blutzuckergeräte im Stand der Technik wie z. B. Accu-Chek Sensor (von Roche Diagnostics) bestehen aus einem Messgerät, in das ein Testelement (Teststreifen, Sensor) eingeführt wird. Der Teststreifen wird mit einem Blutstropfen in Kontakt gebracht, der zuvor mittels einer Stechhilfe aus der Fingerbeere oder einem anderen Körperteil gewonnen wurde. Die zahlreichen Systemkomponenten (Lanzette, Stechhilfe, Teststreifen und Messgerät) benötigen viel Platz und bedingen ein relativ komplexes Handling. Inzwischen gibt es auch Systeme mit einer höheren Anzahl von integrierten Funktionen und damit einer einfacheren Handhabung. Hierzu gehören beispielsweise das AccuChek Compact (von Roche Diagnostics), das Glucometer Dex (von Bayer Diagnostics) sowie das Soft-Sense (von Medisense). Bei den beiden erstgenannten Systemen werden die Teststreifen im Messgerät magaziniert und für die Messung zur Verfügung gestellt.

Ein nächster Schritt der Kompaktheit ist durch die Wahl der Geometrie von Stechelementen und analytischen Hilfsmitteln (Disposable) zu erreichen. Durch geeignete Kombination von Stechvorgang und sensorischer Analytkonzentrations-Erfassung auf einem Teststreifen lässt sich beispielsweise der Bedienablauf deutlich vereinfachen. Hierfür gibt es im Stand der Technik folgende Beispiele:
In der Patentanmeldung US 2004/0039303A1 wird ein integriertes System beschrieben, in dem sowohl mehrere Testelemente als auch mehrere Stechelemente in einem Gerät bevorratet werden. Der zu stechende Körperteil wird an einer Öffnung des Gerätes positioniert, durch die sowohl eingestochen wird als auch Flüssigkeit entnommen wird. Die Stechelemente und Testelemente sind in einem integrierten Dispo unterhalb der Öffnung angeordnet. Durch diese Anordnung muss das Stechelement das Testelement durchstoßen, sodass direkt vom Stechort vom Testelement Flüssigkeit aufgenommen werden kann. Ein Nachteil des Durchstechens des Testelementes ist der potenzielle Eintrag von Material in den Körper. Außerdem weist das Testelement nach dem Durchstechen eine potenzielle Leckstelle auf, durch die Flüssigkeit in das System eindringen kann und das System kontaminieren kann. Außerdem kann die Nadel beim Durchstoßen des Testelementes unscharf werden und dadurch mehr Schmerz verursachen.

In der Patentschrift DE 10208575 wird ein integriertes Blutanalysegerät beschrieben, das mehrere Stechelemente und mehrere Testelemente beinhaltet. Die Testelemente bzw. Stechelemente sind kreisförmig auf zwei Trägern angebracht, die übereinander angeordnet sind. Die Stechelemente bzw. Testelemente werden in zwei verschiedene Arbeitspositionen gebracht, so dass der Benutzer zum Stechen eine andere Öffnung benutzen muss als beim Bluttransfer. Dieses System weist den Nachteil auf, dass der Patient einen Positionswechsel des zu stechenden Körperteils vornehmen muss und dabei das Gehäuse und die Umgebung mit Blut kontaminieren kann. Bei dem Positionswechsel ist zudem eine genaue Positionierung des gestochenen Körperteils auf die zweite Öffnung nötig, um genügend Blut auf das Testelement zu applizieren. Durch die unterschiedlichen Positionierungen von Stechelement und Testelement ist zudem ein getrennter Antrieb für die beiden Elemente nötig.

Aus den Nachteilen des Standes der Technik ergibt sich die Aufgabe, ein Gerät zur Blutentnahme bereitzustellen, das möglichst schmerzarm und kontaminationsarm ausgestaltet und dabei möglichst kompakt, leicht handhabbar ist.

Zur Lösung der Aufgabe wird ein System zur Analyse einer Körperflüssigkeit beschrieben, beinhaltend mindestens ein Stechelement, bevorratet in einem ersten Magazin, mindestens ein Testelement, bevorratet in einem zweiten Magazin, ein Gehäuse, das mindestens das erste Magazin und das zweite Magazin zumindest zum Teil umgibt und eine Öffnung zur Auflage eines Körperteils aufweist, wobei mindestens ein Bereich des ersten und ein Bereich des zweiten Magazins unterhalb der Öffnung im Gehäuse angeordnet sind und dass eines der Magazine mindestens zu einem Teil zwischen Öffnung und dem anderen Magazin angeordnet ist, wobei die beiden Magazine mindestens zu einem Teil bevorzugter weise unter der Öffnung überlappen.

Das System besitzt folglich ein Gehäuse mit einer Öffnung. Erfindungsgemäß können sowohl das Stech- als auch das Testelement zu dieser Öffnung bewegt werden. Dadurch wird sowohl der Stechvorgang als auch die Aufnahme der Flüssigkeit mit Hilfe des Testelements durch eine einzige Öffnung ermöglicht. Mindestens eine Aussparung in mindestens einem Magazin erlaubt ein Hindurchbewegen eines Elementes des der Öffnung abgewandten Magazins durch die Ebene des anderen Magazins, obwohl die beiden Magazine untereinander unter der Öffnung zum Teil überlappend angeordnet sind. Hierdurch wird sowohl das Stechen des Körperteils, das auf der Öffnung aufliegt ermöglicht als auch das Aufnehmen der austretenden Körperflüssigkeit mit dem Testelement an der gleichen Öffnung, ohne dass dabei ein Element des einen Magazins durch ein Element des anderen Magazins geführt oder gestochen werden muss. Als Element ist ein Teil des Magazins gemeint, das im Falle des ersten Magazins das Mittel zum Stechen enthält und im Fall des zweiten Magazins das Mittel zur Probeaufnahme mit den entsprechenden Reagenzien. Wenn der Begriff Element nicht näher spezifiziert ist, kann er alle Mittel zum Stechen wie Lanzetten, Messer, Nadel, Lanzetten mit Probeaufnahme (z.B. Microsampler) usw. bzw. alle Formen von Testelementen bedeuten. Da die Magazine zum Teil überlappend unter der Öffnung angeordnet sind, befindet sich das eine Magazin zwischen der Öffnung und dem anderen Magazin. Dieses Magazin, das sich zwischen der Öffnung und dem anderen Magazin befindet, wird im Folgenden als mittleres Magazin und das jeweils andere Magazin als unteres Magazin bezeichnet. Es ist dabei nicht festgelegt, ob sich das erste Magazin, mit den Stechelementen oder das zweite Magazin mit den Testelementen zwischen Öffnung und dem jeweils anderen Magazin befindet.

Die Aussparung, die mindestens in das mittlere Magazin eingebracht ist, ermöglicht es, ein Element des unteren Magazins durch die Ebene des mittleren Magazins hindurch zu führen, ohne dass das Element durch ein Teil eines Elementes des anderen Magazins geführt wird. Dabei kann die Aussparung unter die Öffnung bewegt werden, in dem das Magazin auf die gleiche Weise bewegt wird wie beim Vorgang des Weitertaktens. Die Aussparung in dem einen Magazin sollte dabei so groß sein, dass ein Element des anderen Magazins möglichst ungestört durch die Aussparung geführt werden kann. Die Aussparung kann jedoch auch größer sein. Bei dem Einstichvorgang und/oder dem Aufnehmen der Flüssigkeit können sich Teile der Magazine berühren. Diese Berührung sollte die Bewegung des Elementes aber nicht so beeinträchtigen, dass das Element in seiner Funktion beeinträchtigt ist. Es ist keine zusätzliche Bewegung des Magazins für diesen Vorgang notwendig, was bedeutet, dass keine zusätzlichen Antriebseinheiten für die Positionierung der Magazine unter die Öffnung nötig sind.

Dass die Elemente der beiden Magazine bei Benutzung nicht durch Material des jeweils anderen Magazins hindurchgeführt werden, hat den Vorteil, dass kein Materialübertrag von einem Element auf das andere stattfindet. Dadurch kann das zu stechende Körperteil nicht durch Material des durchstochenen Elementes kontaminiert werden, wie dies im Stand der Technik (US 2004/0039303 A1) durch das Durchstoßen des Stechelementes durch das Testelement passieren kann. Außerdem braucht der Benutzer für den Stechvorgang und die Flüssigkeitsapplikation auf das Testelement das gestochene Körperteil nicht von der Öffnung wegzubewegen. Hierdurch wird zusätzlich die Gefahr einer Kontamination des Gerätes mit Körperflüssigkeit verringert.

Weiterhin können die beiden Magazine in einer bevorzugten Ausführungsform komplett überlappen. Stechelemente und Testelemente sind dann konzentrisch angeordnet. In dieser bevorzugten Ausführungsform ist das Weitertakten der Magazine vorteilhafter Weise mit nur einem Antrieb möglich. Geeignete Antriebe sind z.B. von Uhren bekannt. Trotz des einen Antriebs können die Magazine unabhängig voneinander bewegt werden. Bevorzugter weise befindet sich in dem mittleren Magazin zwischen jedem Element eine Aussparung. Durch die Verwendung nur eines Antriebs ist es möglich, die Bewegung der beiden Magazine exakt aufeinander abzustimmen. Bevorzugter weise können die beiden Magazine synchron bewegt werden. Durch diese mindestens eine Aussparung kann das Element des anderen Magazins ungehindert benutzt werden. Die Aussparung kann bei Benutzung des Systems durch den gleichen Mechanismus unter die Öffnung gebracht werden, wie das Weitertakten des Magazins.

In einer weiteren bevorzugten Ausführungsform können beide Magazine alternierend zu den Elementen eine Aussparung besitzen. Auf diese Weise sind die Elemente des einen Magazins alternierend zu den Elementen des anderen Magazins angeordnet, so dass durch gleichzeitiges Weitertakten der beiden Magazine jeweils ein Element unter der Öffnung zur Benutzung bereitgestellt werden kann. Dabei können die Magazine sehr Raum sparend zueinander angeordnet werden und trotzdem kann eine Benutzung der Stech- und Testelemente durch eine Öffnung gewährleistet werden. Die Handhabung eines solchen integrierten Systems ist sehr einfach, da der Benutzer sein zu stechendes Körperteil auf die Öffnung legt und danach sowohl der Stechvorgang als auch der Testvorgang ohne weitere Handhabungsschritte ablaufen können. Trotz der kompakten Anordnung der Magazine besitzt die getrennte Magazinierung von Stechelementen und Testelementen den Vorteil, dass die Stechelemente unabhängig von den Testelementen hergestellt werden können. Dies hat besondere Vorteile, wenn unterschiedliche Materialien für die verschiedenen Bauteile wie beispielsweise Elemente, Halterungen und/oder Magazingehäuse verwendet wird. Auf dies Art und Weise kann auch der Sterilisationsprozess, der für die Stechelemente notwendig ist, getrennt von den Testelementen durchgeführt werden. Die Magazine können also vor dem Einsatz in das System vollständig separat voneinander gehandhabt werden und erst nach ihrer Herstellung oder bei Benutzung zusammengeführt oder einzeln in das System eingesetzt werden.

Unter Magazin wird im Rahmen der Erfindung eine Anordnung von mehr als einem Element, in diesem Beispiel von Stechelementen bzw. Testelementen verstanden, die in einer Haltestruktur bevorratet sind. Diese Haltestruktur kann beispielsweise ein Gehäuse sein, in dem mehrere Elemente voneinander getrennt bevorratet sind, wie dies beispielsweise in einem Stapelgehäuse der Fall ist. Alternativ kann die Haltestruktur ein Grundkörper sein, an dem die Elemente (wie Stech- bzw. Testelemente) eines Magazins befestigt sind. Die einzelnen Elemente können dabei eine Folie besitzen, die einen Schutz des Stech- oder Testelementes gegen die Umgebung darstellen kann und außerdem als Sterilschutz dienen kann. Das Magazin ist folglich nicht automatisch von einem Gehäuse oder einer Folie umgeben oder durch eine Folie oder Membran gegen die Umgebung abgeschirmt. Es sind auch Anordnungen denkbar, in denen die beiden Magazine zusammen in ein Gehäuse oder in eine Folie eingebracht sind. Zusätzlich können die Magazine mindestens zu einem Teil einen Sterilschutz, z.B. in Form einer Folie aufweisen. In einer bevorzugten Ausführungsform weisen mindestens die Lanzettenspitzen einen Sterilschutz auf.

Die erste Haltestruktur mit dem mindestens einen Stechelement und die zweite Haltestruktur mit dem mindestens einen Testelement können einstückig sein oder aus verschiedenen Elementen bestehen. Dabei können die Haltestrukturen aus verschiedenen Materialien bestehen, wie Metall, Keramik oder Kunststoff. In einer bevorzugten Ausführungsform bestehen die beiden Haltestrukturen aus einem Stück, das beispielsweise aus Metall gefertigt ist. In einer bevorzugten Ausführungsform bildet die Haltestruktur das Magazin, da sie keine weitere Umhüllung wie ein Gehäuse aufweist.

Bei der Herstellung eines einstückigen Stechelementmagazins ist darauf zu achten, dass die Lanzettenspitze dazu geeignet sein soll, in die Haut eines Körperteils einzustechen. Die Lanzettenspitze weist einen distalen Bereich auf, der in einem spitzen Punkt mündet. In dem Magazin für Stechelemente sind mindestens 2 Lanzetten bevorratet, es können sich jedoch mehr als 100 Lanzetten in dem ersten Magazin befinden. In einer bevorzugten Ausführungsform befinden sich bis zu 50 Lanzetten in dem ersten Magazin. Auch das zweite Magazin beinhaltet in einer bevorzugten Ausführungsform mehr als ein Testelement. Es können sich mehr als 100 Testelemente in dem zweiten Magazin befinden. Bevorzugter weise befinden sich 50 Testelemente in dem zweiten Magazin.

Die beiden Magazine weisen eine Kreisscheibenform auf. Diese Kreisscheibenform wird im Falle des ersten Magazin als Lanzettenrad und im Falle des zweiten Magazins als Testelementrad bezeichnet.

Die kreisförmige Ausbildung des ersten und zweiten Magazins in Form eines Lanzettenrads bzw. eines Testelementrads ist bevorzugt. In dieser Ausführungsform besitzen die Räder von einander getrennt angeordnete Arme (Lanzettenarm bzw. Testelementarm) an deren Spitze entweder eine Lanzettenspitze oder ein Nachweisbereich angeordnet ist. In einer bevorzugten Ausführungsform sind die Radachsen des Lanzettenrads und des Testelementrads senkrecht zur Öffnungsebene der Öffnung angeordnet. Durch die Überlappung der Räder und ihrer Ausrichtung zur Öffnung innerhalb des Systems weist der Lanzettenarm in seiner Ausrichtung nicht in Richtung der Öffnung des Systems. Ist die Lanzettenspitze parallel zum Lanzettenarm ausgerichtet, so ist in einer bevorzugten Ausführungsform das Abknicken der Lanzette vor Benutzung vorgesehen. Dieses Abknicken führt zu einer Ausrichtung der Lanzettenspitze orthogonal zum Lanzettenarm und die Lanzettenspitze weist in Richtung Öffnung und kann so beim Einstichvorgang in Richtung Öffnung bewegt werden. Dabei kann durch ein Regelelement in der Knickvorrichtung die Stelle variiert werden, an der die Lanzettenspitze abgeknickt wird. Hierdurch kann die Stechtiefe eingestellt werden, da der nicht abgeknickte Bereich der Lanzettenspitze bzw. des Lanzettenarms als Anschlagelement beim Einstich in die Haut dienen kann. Alternativ kann die Lanzettenspitze bereits direkt bei oder nach der Herstellung des Lanzettenrades orthogonal zum Lanzettenarm ausgerichtet werden, wie dies im Stand der Technik in dem US Patent 4,794,926 beschrieben ist.

Alternativ zur Ausführung als Stechelementrad und Testelementrad mit federnden Ärmchen sind auch Scheiben zu verwenden, die als Ganzes zur Öffnung bewegt werden. Hierbei ist vorteilhaft, wenn die bewegten Massen nicht zu groß sind, da dies entsprechend starke und große Antriebe besonders für die schnelle Stechbewegung erfordern würde. Diese bevorzugte Ausführungsform eignet sich daher für besonders kleine und damit vorteilhafte Ausführungen von Test- und Stechelementmagazinen.

Die Lanzettenarme des Lanzettenrades können bevorzugter weise entweder aus der kreisförmigen Struktur ausgestanzt und/oder heraus geätzt werden. Es sind jedoch auch andere im Stand der Technik bekannte Verfahren zur Herstellung von Lanzetten möglich. Sowohl beim Stanzen als auch beim Ätzen können die Lanzettenspitzen in einem Schritt ausgebildet werden. Der Lanzettenarm ist federnd mit dem Lanzettenkörper verbunden, sodass sich der Lanzettenarm aus der Kreisebene des Lanzettenrandes herausbewegen lässt. Diese Auslenkung ist sowohl unterhalb der Lanzettenkörperebene als auch oberhalb der Lanzettenkörperebene möglich. Diese Flexibilität des Lanzettenarms kann durch verschiedene Methoden erreicht werden. Eine Möglichkeit ist die Herstellung des Lanzettenrades aus sehr dünnem Material (z. B. Metall oder Kunststoff). Durch die niedrige Dicke des Lanzettenrades lassen sich die Lanzettenarme leicht biegen. Eine weitere Methode, den Lanzettenarm flexibel zum Lanzettenkörper zu gestalten, ist das Einbringen einer Prägung an der Übergangsstelle von Lanzettenarm zum Lanzettenkörper. Die Prägung kann eine Verjüngung des Materials sein, die beispielsweise durch Stanzen oder Ätzen dort eingebracht werden kann.

Zur hygienischen Verwendung des Systems kann die Lanzette mindestens im Spitzenbereich durch einen Sterilschutz geschützt sein. Bevorzugter weise ist die Lanzette über den ganzen Lanzettenkörper mit einer Folie als Sterilschutz bedeckt. Dieser Sterilschutz kann beispielsweise aus einer Polymerschicht bestehen oder aus einer Polymerkappe, die nach der Produktion der Lanzettenstruktur aufgebracht wird. Der Sterilschutz kann auch aus dünnen Folien bestehen, die die Lanzettenspitze oben und unten umgeben und beim Knicken durchbrochen werden. Diese Folien werden zweckmäßigerweise durch einen kleinen Rahmen, der um die Lanzettenspitze herum geführt wird, gehalten. Der Sterilschutz wird beim Einstichvorgang (beispielsweise durch einen Teil der Öffnung) durchstochen oder beim Aufwenden der Schwellenkraft auf die Lanzettenspitze von der Lanzettenspitze beim Abknicken zerstört und legt die Lanzettenspitze frei. Alternativ kann der Sterilschutz vor Benutzung der Lanzette entfernt werden. Bevorzugter weise wird der Sterilschutz hierbei im Ganzen entfernt. Beim Entfernen des Sterilschutzes sollte die Lanzettenspitze nicht direkt berührt werden, um nicht die Sterilität zu beeinträchtigen.

In einer bevorzugten Ausführungsform befindet sich an verschiedenen Stellen im Gehäuse und/oder an mindestens einem der Magazine Trockenmittel, das bewirkt, dass die Testelemente einer gegenüber der Umgebungsluft erniedrigten Luftfeuchte ausgesetzt sind. Dadurch kann die Haltbarkeit der Testelemente erhöht werden. Um die Feuchtigkeit im Gehäuse annähernd konstant zu halten, kann das Gehäuse eine Abdichtung gegen die Umgebung besitzen, sodass sowohl das Magazin für die Stechelemente als auch das Magazin für die Testelemente gegenüber der Umgebungsfeuchtigkeit abgeschirmt sind. Hierdurch wird eine Gehäuseatmosphäre erreicht, die eine erniedrigte Feuchtigkeit gegenüber der Umgebungsatmosphäre aufweist. Bevorzugter weise besitzt die Gehäuseöffnung dann einen Dichtungsmechanismus, der bei Benutzung geöffnet werden kann und anschließend wieder verschlossen wird.

Der Vorteil bei der Verwendung von zwei getrennten Halterungen bzw. Magazinen für Lanzetten und Testelementen, ist die Möglichkeit zur getrennten Herstellung der anders gearteten Elemente. Hierbei treten keine Unverträglichkeiten von Prozessen, wie beispielsweise der Sterilisation für die verschiedenen Elemente auf.

Aufgrund der Möglichkeit, eine ähnliche Geometrie für die beiden Magazine zu wählen, ist es jedoch möglich sie miteinander zu koppeln bzw. sie gleichartig zu aktuieren und sie trotzdem einzeln anzusteuern. Es ist jedoch auch möglich die beiden Magazine so mit einander zu koppeln, dass sie gleichzeitig bewegt werden. So können die beiden Magazine in ein Gehäuse eingebaut sein, das zusammen als Disposable dienen kann.

Das Blut kann auf verschiedene Komponenten hin untersucht werden, wie es im Stand der Technik bekannt ist. Zum Beispiel kann die Analyse auf Blutbestandteile wie Hämatokrit, Glucose, Cholesterin, Koagulation, Eisen und andere gerichtet sein. Zur Analyse können unterschiedliche Methoden zur Anwendung kommen. So können beispielsweise elektrochemische Nachweisreaktionen benutzt werden, aber auch optische (z.B. Reflektion, Absorption, Fluoreszenz, Raman-Spektroskopie) oder magnetische Nachweisreaktionen. Vorzugsweise weist das mindestens eine Testelement mindestens eine Reagenzschicht auf, welche ausgestaltet ist, um bei Kontakt mit dem mindestens einen nachzuweisenden Analyten mindestens eine Eigenschaft, insbesondere eine optische und/oder elektrochemische Eigenschaft zu ändern. Typischerweise wird die Flüssigkeit mit einem Testsystem in Kontakt gebracht, wobei eine Reaktion zwischen einem Testelement und der Flüssigkeit stattfindet. So beruht beispielsweise die Detektion mittels eines optischen Testelements auf einer Farbreaktion zwischen Flüssigkeit und Nachweisreagenz. Beispiele für diese Reaktionen sind in den US Patenten 3,802,842, 4,061,468 und 4,490,465 beschrieben.

Zum Antrieb des Stechelementes bzw. des Testelementes können ballistische oder kulissengeführte Mechanismen benutzt werden, die aus dem Stand der Technik bekannt sind, wie z.B. in DE19604156, EP0565970, US5318584 oder US4924879 beschrieben. Eine bevorzugte Ausführungsform für den Antrieb des Stechelementes bzw. Testelementes ist die freie Bewegung des Elementes nach Kraftübertragung durch das Antriebselement, wie beispielsweise einem Stößel. In dieser Ausführungsform wird ein Impuls von dem Antriebselement auf das Stechelement bzw. das Testelement übertragen und das Element bewegt sich ohne weitere Führung durch das Antriebselement in Richtung Gehäuseöffnung. Die Bewegung der Lanzette kann dabei durch zusätzliche Elemente am Gehäuse geführt werden.

Das Stechelement bzw. das Testelement können alternativ durch Greifelemente ergriffen werden und in Richtung Öffnung bewegt werden.

Der Antrieb kann in seinem zeitlichen Verlauf variiert werden. So kann beispielsweise bei geführten Einstichprofilen der Lanzette, die Bewegung zur Öffnung schneller erfolgen als die Bewegung von der Öffnung weg. Dies ist besonders bevorzugt, wenn die Lanzette zur Flüssigkeitsaufnahme geeignet ist. Die Bewegung des Antriebelements kann auch während der Einstichbewegung bzw. während der Austrittbewegung von der Öffnung weg gestoppt werden.

In einer weiteren Ausführungsform kann die Antriebseinheit dazu geeignet sein, das Stechelement oder Testelement sowohl aus der Position im unbenutzten Zustand in Richtung Öffnung zu bewegen als auch in die entgegen gesetzte Richtung. In einer bevorzugten Ausführungsform, in der die Haltestruktur in Form einer Disk mit sich radial nach außen oder innen erstreckenden, federnd gelagerten Armen ausgestaltet ist, wird dabei die Federkraft des flexibel ausgestalteten Stechelementarms oder des Testelementarms dazu benutzt, den Arm des Elementes in die der Öffnung entgegen gesetzten Richtung zu spannen und den gespannten Arm, wenn notwendig durch Lösen der Spannung zur Öffnung schnellen zu lassen. Diese Bewegungsrichtung des Spannens des Armes kann weiterhin dazu verwendet werden, ein Stechelement mit einem Testelement in Berührung zu bringen, um beispielsweise Flüssigkeit von der Lanzettenspitze bzw. Microsamplers auf das Testelement zu übertragen.

In einer bevorzugten Ausführungsform werden Stechelement und Testelement mit einer Antriebseinheit bewegt. Die Antriebseinheit kann dabei andere Bewegungsprofile beim Antrieb des Stechelements aufweisen als beim Antrieb des Testelements. Dies kann durch eine elektronische Regeleinheit in der Antriebseinheit gewährleistet werden.

Die Veränderung der Eigenschaften des Testelementes nach Aufgabe der Probe kann beispielsweise durch optische Detektoren erfolgen. Dies sind herkömmliche Geräte, wie Photodioden, CMOS-Geräte oder CCD-Kameras, wie sie aus dem Stand der Technik bekannt sind.

### Figurenbeschreibung

**Figur 1**: Schematische Darstellung eines Systems zur Analyse einer Körperflüssigkeit mit einer Öffnung, einem Magazin für Stechelemente und einem Magazin für Testelemente, die nebeneinander angeordnet sind (Aufsicht).
**Figur 2**: Schematische Darstellung des Systems wie in Figur 1 in Seitenansicht mit Knickvorrichtung, Antrieb und Detektionseinheit.
**Figur 3**: Schematische Darstellung der beiden Magazine für Stechelemente und Testelemente, die übereinander angeordnet sind.
**Figur 4a**: Schematische Darstellung einer Vorrichtung zum Knicken der Lanzettenspitze in Form einer Zange.
**Figur 4b**: Schematische Darstellung der Vorrichtung zum Knicken der Lanzettenspitze mit einem runden Zangenende.

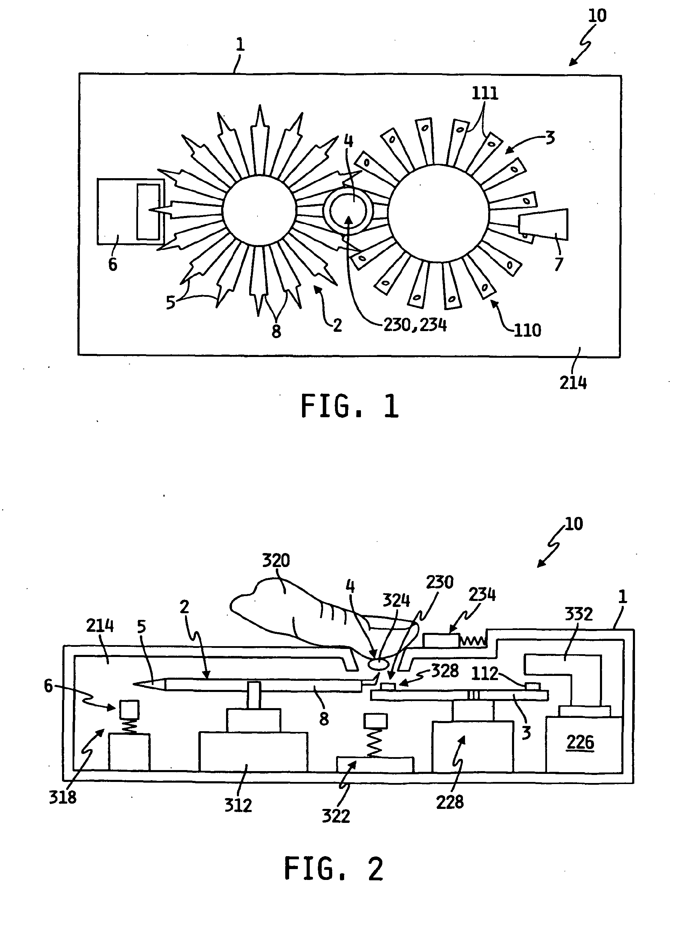

In den Figuren 1 und 2 ist das System (10) und die Funktionsweise des Systems (10) zur Analyse einer Körperflüssigkeit schematisch dargestellt. Figur 1 zeigt eine Aufsicht auf das System und Figur 2 zeigt eine Seitenansicht davon. Im Betrieb wird zunächst eine zu verwendende Lanzettenspitze (5) in eine Knickstation (6) gedreht, in der sich ein Knickelement (hier nicht gezeigt) befindet. In dieser Knickstation (6) wird mittels eines Federmechanismus (318) die zu verwendende Lanzette (5) um ca. 90° nach oben (in Figur 2) geknickt. Anschließend wird mittels des Antriebs (312) das Lanzettenrad (2) um 180° gedreht, bis die nach oben geknickte Lanzettenspitze (5) unterhalb der Applikationsöffnung (4) im Gehäuse (1) steht. Die Applikationsöffnung (4) ist in diesem Ausführungsbeispiel als Konus ausgeführt und kann vor und nach Gebrauch mit einem Dichtelement (234) verschlossen sein. Das Dichtelement (234) kann mit dem Finger (320) des Patienten beiseite geschoben und dadurch die Applikationsöffnung (4) freigelegt werden. Der Finger (320) verschließt diese Applikationsöffnung (4) dabei gleichzeitig. Durch Aufpressen des Fingers (320) auf die Applikationsöffnung (4) bildet die Hautpartie des Fingers (320) im Bereich der Applikationsöffnung (4) eine ins Innere des Gehäuses (1) gewölbte Beule. Mittels eines - Federmechanismus (322) wird beim Auslösen des tragbaren Messsystems (210) die sich unterhalb der Applikationsöffnung (4) befindliche Lanzettenspitze (5), welche nach oben gebogen ist, beschleunigt und perforiert die Hautpartie des Fingers (320) innerhalb der Applikationsöffnung (4). Dadurch bildet sich ein Blutstropfen (324).

Das in diesem Ausführungsbeispiel gemäß den Figuren 1 und 2 kreisscheibenförmig ausgebildete Testelementrad (3) ist in Kreisscheiben-Sektoren, die Testelementarme (111) unterteilt. Zwischen diesen Testelementarmen (111) befinden sich Aussparungen (103) (in Figur 2 nicht gezeigt). Zum Zeitpunkt des Einstichs der Lanzettenspitze (5) in die Haut befindet sich das Testelementrad (3) in einer Konfiguration, sodass sich eine Aussparung (103) unter der Öffnung befindet, damit das ungehinderte Antreiben des Lanzettenarmes (8) möglich wird. Jeder dieser Testelementarme (111) beinhaltet ein Testelement (110) mit einem Nachweisbereich (112). Jeder dieser Nachweisbereiche (112) weist eine Reagenzschicht (328) auf, welche, wie oben beschrieben, auf die Anwesenheit und/oder Konzentration des Analyten in der flüssigen Probe (Blutstropfen (324)) reagiert. In diesem Ausführungsbeispiel gemäß den Figuren 1 und 2 handelt es sich bei dieser Reagenzschicht (328) beispielsweise um eine bei Anwesenheit von Glukose eine Farbreaktion durchführende Reagenzschicht (328), d. h. eine Reagenzschicht (328), welche durch Reaktion mit Glukose ihre Farbe und/oder ihre Fluoreszenzeigenschaften ändert.

Nach Perforation der Hautpartie (wie oben beschrieben) kann das Testelementrad (3) mit Hilfe des Antriebs (228) soweit gedreht werden, dass der Testelementarm (111) durch den - Federmechanismus (322) kurzfristig nach oben, hin zur Applikationsöffnung (4), gebogen werden kann. Dadurch wird der Blutstropfen (324) auf die Reagenzschicht (328) des sich in der Applikationsposition (230) befindlichen Nachweisbereich (112) aufgebracht, und es kann die beschriebene Reaktion erfolgen.

Anschließend wird mittels des Antriebs (228) das Testelementrad (3) um 180° gedreht, so dass der Nachweisbereich (112), auf welches der Blutstropfen (324) appliziert wurde, unterhalb eines optischen Auslesers (332) platziert ist. Dieser optische Ausleser (332) führt beispielsweise eine einfache optische Messung oder eine Fluoreszenzanregungs-Messung durch. Der optische Ausleser (332) ist mit der elektronischen Auswertungsvorrichtung (226) verbunden, die beispielsweise einen Mikroprozessor, Bedienelemente, Displays, Datenspeicher oder ähnliches umfassen kann.

Das voll integrierte tragbare Messsystem (10) gemäß dem Ausführungsbeispiel in den Figuren 1 und 2 zeigt besonders deutlich die Vorteile der erfindungsgemäßen Lösung. Die Anordnung gewährleistet den Antrieb von Lanzettenspitze (5) und Testelementarm (8) mit nur einem Antrieb (322). In dieser speziellen Ausführungsform ist das Innere des Gehäuses (1) mit der einen Öffnung (4) durch eine Dichtung (234) vor Umgebungseinflüssen wie Feuchtigkeit geschützt. Bei Benutzung wird diese Dichtung (234) durch das zu stechende Körperteil, in diesem Fall einem Finger (320) beiseite geschoben, um die Öffnung (4) des Gehäuses (1) zur Benutzung frei zu legen. Hierbei ist die Reagenzschicht (328) und die Lanzettenspitze (5) der Gehäuseinnenatmosphäre (214) ausgesetzt. Eine separate Versiegelung der einzelnen Nachweisbereiche (112) ist weder erforderlich noch zweckmäßig. Müsste zunächst eine Versiegelung von den einzelnen Reagenz schichten (328) entfernt werden, so würde dies einen zusätzlichen mechanischen Aufwand erfordern, welcher sich wiederum nachteilig auf den Energiebedarf sowie auf den Bauraum auswirken würde, da weitere mechanische Vorrichtungen und Betätigungselemente erforderlich wären. Wiederum kann beispielsweise ein Trocknungsmittel in das hier kreisscheibenförmig ausgestaltete Testelementrad (3) integriert sein.

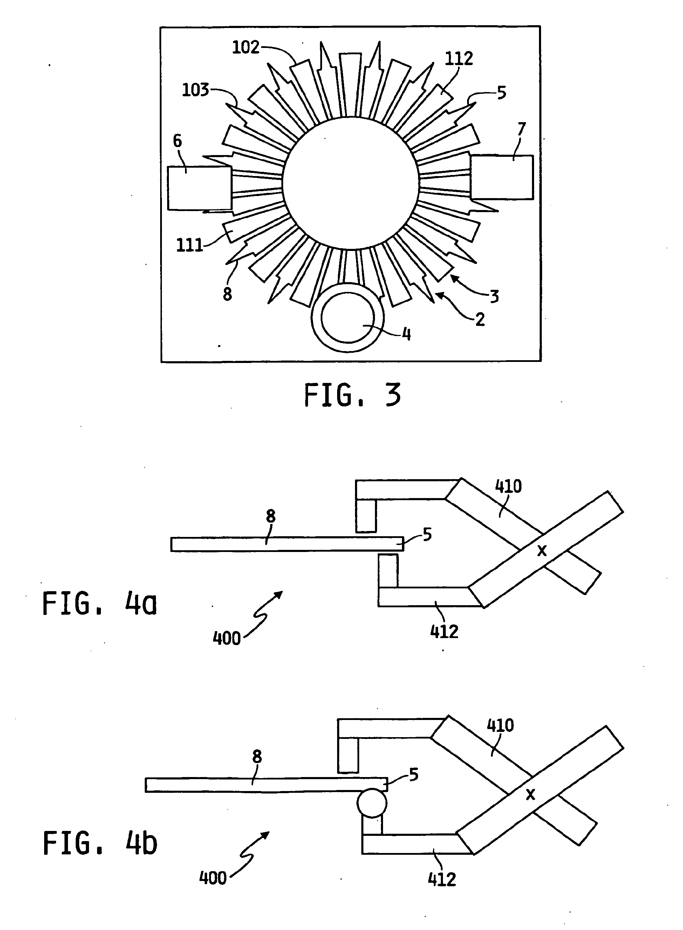

In Figur 3a ist eine weitere Möglichkeit der Anordnung der beiden Magazine, des Lanzettenrad (2) und des Testelementrad (3) aufgezeigt. Die beiden Magazine sind kreisförmig ausgebildet und weisen zwischen ihren Elementen Aussparungen auf. Hierbei überlappen die beiden kreisförmigen Magazine (2) und (3) vollständig. Aufgrund der Aussparungen (102) in dem Lanzettenrad (2) und Aussparungen (103) in dem Testelementrad (3) können die Lanzettenspitzen (5) und die Nachweisbereiche (112) alternierend angeordnet werden, und so kann eine gegenseitige Behinderung der Elemente bei Benutzung verhindert werden. Diese Anordnung der Magazine weist den Vorteil auf, dass für das Weitertakten und/oder für den Antrieb der Elemente jeweils nur eine Antriebseinheit benötigt wird. Selbst bei Verwendung nur eines Antriebs zum Weitertakten der Magazine ist es möglich, beide Magazine unabhängig voneinander zu bewegen. Da die Magazine (2) und (3) sehr dünn gearbeitet werden können, ist es unerheblich, ob sich das Lanzettenrad (2) oder das Testelementrad (3) näher an der Öffnung befindet. In dieser vollständig überlappenden Geometrie ist es möglich das System wie in Figur 2 dargestellt so abzuwandeln, dass nur noch ein Antrieb sowohl für den Lanzettenarm (8) als auch für den Testelementarm (111) nötig ist. Auf diese Weise kann das System weiterhin verkleinert werden. Die Öffnung (4) ist in diesem Beispiel nicht zwischen der Knickstelle (6) und dem Detektor (7) angeordnet, da Knickstelle (6) und Detektor (7) näher zueinander angeordnet sind als in Figur 1.

In Figur 4a ist eine Vorrichtung zum Abknicken der Lanzettenspitze (5) vor dem Einstich gezeigt. Dies ist eine zangenähnliche Struktur 400, die zwei Hebel (410) und (412) aufweist, die eine unterschiedliche Länge aufweisen. Der Hebel (410) kann zur Fixierung des Lanzettenarmes (8) benutzt werden, während der Hebel (412) eine Schwellenkraft in Richtung des anderen Hebels (410) auf die Lanzettenspitze (5) ausübt, um diese aus der Ebene des Lanzettenarmes (8) herauszuknicken. Je nach Ausmaß der Krafteinwirkung auf die Lanzettenspitze (5), kann die Lanzettenspitze(5) bis zu 90° aus der Ebene des Lanzettenarmes (8) heraus geknickt werden.

In Figur 4b ist eine Abwandlung dieser Zangenvorrichtung aus Figur 4a gezeigt, in der die Spitze mindestens eines Hebels, z.B. Hebel (412) rund ausgestaltet ist, um eine Verletzung der Lanzettenspitze (5) beim Abknicken zu vermeiden. Aus dem Stand der Technik sind viele weitere Vorrichtungen zum Abknicken dünner Metallbleche bekannt.

## Patentansprüche

1. System zur Analyse einer Körperflüssigkeit, beinhaltend
• mindestens ein Stechelement, bevorzugt eine Lanzette, bevorratet in einem ersten Magazin (2),
• mindestens ein Testelement (110) mit einem Nachweisbereich (112), bevorratet in einem zweiten Magazin (3),
• ein Gehäuse, das mindestens das erste Magazin (2) und das zweite Magazin (3) zumindest zum Teil umgibt und eine Öffnung zur Auflage eines Körperteils aufweist, wobei mindestens ein Bereich des ersten und ein Bereich des zweiten Magazins unterhalb der Öffnung (4) im Gehäuse derartig angeordnet sind, dass eines der Magazine mindestens zu einem Teil zwischen Öffnung (4) und dem anderen Magazin angeordnet ist, wobei die beiden Magazine eine Kreisscheibenform aufweisen und mindestens zu einem Teil überlappen, und dass sowohl das Stechelement als auch das Testelement in Richtung der Öffnung bewegt werden können, um mit dem Stechelement das Stechen des Körperteils, wenn dieses auf der Öffnung aufliegt, zu ermöglichen, und um an der gleichen Öffnung die austretende Körperflüssigkeit mit dem Testelement aufzunehmen, **dadurch gekennzeichnet, dass**
das erste und/oder zweite Magazin mindestens eine Aussparung (102, 103) aufweist die eine Bewegung des Test- bzw. Stechelementes zur Aufnahme von Körperflüssigkeit bzw. zum Stechen eines Körperteils durch die Ebene des anderen Magazins hindurch zur Öffnung hin ermöglicht.

2. System nach Anspruch 1, **dadurch gekennzeichnet, dass** das mindestens eine Stechelement und das mindestens eine Testelement nacheinander zur Öffnung bewegt wird.

3. System nach einem der vorherigen Ansprüche, **dadurch gekennzeichnet, dass** die Magazine durch den gleichen Antrieb weitergetaktet werden.

4. System nach einem der vorherigen Ansprüche, **dadurch gekennzeichnet, dass** das mindestens eine Stechelement und das mindestens eine Testelement durch den gleichen Antrieb zur Öffnung bewegt wird.

5. System nach einem der vorherigen Ansprüche, **dadurch gekennzeichnet, dass** das mindestens eine Stechelement und/oder das mindestens eine Testelement an einem federnden Träger angebracht ist, sodass das Stechelement bzw. das Testelement sowohl nach oben zur Öffnung hin als auch nach unten von der Öffnung weg ausgelenkt werden kann.

6. System nach einem der vorherigen Ansprüche **dadurch gekennzeichnet, dass** das Stechelement zur Flüssigkeitsaufnahme ausgestaltet ist.

7. System nach Anspruch 6 **dadurch gekennzeichnet, dass** das mindestens eine Stechelement und das mindestens eine Testelement mit einander in Berührung gebracht werden.

8. System nach einem der vorherigen Ansprüche, **dadurch gekennzeichnet, dass** das System eine Detektionseinheit zur Detektion des Testelementes aufweist.

9. System nach einem der vorherigen Ansprüche, **dadurch gekennzeichnet, dass** das System eine Auswerteeinheit aufweist.

10. System nach einem der vorherigen Ansprüche, **dadurch gekennzeichnet, dass** das System ein Display aufweist.

11. System nach einem der vorherigen Ansprüche, **dadurch gekennzeichnet, dass** das mindestens eine Stechelement zur Benutzung abgeknickt wird.

12. System nach Anspruch 11, **dadurch gekennzeichnet, dass** die Stelle an der das Stechelement abgeknickt wird, vor Benutzung ausgewählt wird.

13. System nach einem der vorherigen Ansprüche, **dadurch gekennzeichnet, dass** das mindestens eine Stechelement einen Sterilschutz aufweist.

14. System nach einem der vorherigen Ansprüche, **dadurch gekennzeichnet, dass** die Öffnung ein Druckstück zur Auflage eines Körperteils auf die Öffnung aufweist.

## Claims

1. System for analysing a body fluid comprising
■ at least one lancing element, preferably a lancet, stored in a first magazine (2),
■ at least one test element (110) with a detection area (112) stored in a second magazine (3),
■ a housing which at least partially surrounds at least the first magazine (2) and the second magazine (3) and has an opening for supporting a body part, wherein at least one area of the first and one area of the second magazine are arranged below the opening (4) in the housing in such a manner that one of the magazines is arranged at least partially between the opening (4) and the other magazine, wherein the two magazines have a disk shape and at least partially overlap, and in such a manner that the lancing element as well as the test element can be moved towards the opening in order to enable the lancing element to lance the body part when it rests on the opening and to enable the test element to pick up the emerging body fluid at the same opening,
**characterized in that**
the first and/or the second magazine has at least one cut-out (102, 103) which enables the test element or lancing element to be moved through the plane of the other magazine towards the opening in order to pick up body fluid or to lance a body part.

2. System according to claim 1, **characterized in that** the at least one lancing element and the at least one test element are moved one after another towards the opening.

3. System according to one of the previous claims, **characterized in that** the magazines are advanced stepwise by the same drive.

4. System according to one of the previous claims, **characterized in that** at least one lancing element and the at least one test element is moved by the same drive towards the opening.

5. System according to one of the previous claims, **characterized in that** the at least one lancing element and/or the at least one test element is attached to a flexible support such that the lancing element or the test element can be deflected upwards towards the opening as well as downwards away from the opening.

6. System according to one of the previous claims, **characterized in that** the lancing element is designed to pick up liquid.

7. System according to claim 6, **characterized in that** the at least one lancing element and the at least one test element are contacted with one another.

8. System according to one of the previous claims, **characterized in that** the system has a detection unit for detecting the test element.

9. System according to one of the previous claims, **characterized in that** the system has an evaluation unit.

10. System according to one of the previous claims, **characterized in that** the system has a display.

11. System according to one of the previous claims, **characterized in that** the at least one lancing element is bent for use.

12. System according to claim 11, **characterized in that** the site at which the lancing element is bent, is selected before use.

13. System according to one of the previous claims, **characterized in that** the at least one lancing element has a sterile protection.

14. System according to one of the previous claims, **characterized in that** the opening has a pressure piece for supporting a body part on the opening.

## Revendications

1. Système destiné à analyser un liquide corporel, comprenant :
- au moins un élément de piqûre, de préférence une lancette, logé dans un premier magasin (2),
- au moins un élément de test (110) comprenant une zone de détection (112), logé dans un second magasin (3),
- un boîtier qui entoure au moins en partie au moins le premier magasin (2) et le second magasin (3) et qui présente une ouverture pour l'appui d'une partie du corps, au moins une partie du premier magasin et une partie du second magasin étant disposées dans le boîtier en dessous de l'ouverture (4) de manière à ce que l'un des magasins soit disposé au moins pour une partie entre l'ouverture (4) et l'autre magasin, les deux magasins présentant une forme de disque et se chevauchant au moins sur une partie, et à ce que aussi bien l'élément de piqûre que l'élément de test puissent être déplacés en direction de l'ouverture afin de permettre, à l'aide de l'élément de piqûre, la piqûre de la partie du corps lorsque celle-ci s'appuie sur l'ouverture et afin de recevoir à la même ouverture, à l'aide de l'élément de test, le liquide corporel sortant,
**caractérisé en ce que**
le premier et/ou le second magasins présentent au moins un évidement (102, 103) qui permet un mouvement de l'élément de test resp. de l'élément de piqûre pour la réception de liquide corporel resp. pour la piqûre d'une partie du corps à travers le plan de l'autre magasin en direction de l'ouverture.

2. Système selon la revendication 1, **caractérisé en ce que** l'au moins un élément de piqûre et l'au moins un élément de test sont déplacés l'un après l'autre vers l'ouverture.

3. Système selon l'une quelconque des revendications précédentes, **caractérisé en ce que** les magasins sont rythmés par le même entraînement.

4. Système selon l'une quelconque des revendications précédentes, **caractérisé en ce que** l'au moins un élément de piqûre et l'au moins un élément de test sont déplacés vers l'ouverture par le même entraînement.

5. Système selon l'une quelconque des revendications précédentes, **caractérisé en ce que** l'au moins un élément de piqûre et/ou l'au moins un élément de test sont placés sur un support à ressort de manière à ce que l'élément de piqûre resp. l'élément de test puisse être dévié aussi bien vers le haut en direction de l'ouverture que vers le bas en s'éloignant de l'ouverture.

6. Système selon l'une quelconque des revendications précédentes, **caractérisé en ce que** l'élément de piqûre est réalisé pour la réception de liquide.

7. Système selon la revendication 6, **caractérisé en ce que** l'au moins un élément de piqûre et/ou l'au moins un élément de test sont mis en contact l'un avec l'autre.

8. Système selon l'une quelconque des revendications précédentes, **caractérisé en ce que** le système présente une unité de détection pour détecter l'élément de test.

9. Système selon l'une quelconque des revendications précédentes, **caractérisé en ce que** le système présente une unité d'évaluation.

10. Système selon l'une quelconque des revendications précédentes, **caractérisé en ce que** le système présente un afficheur.

11. Système selon l'une quelconque des revendications précédentes, **caractérisé en ce que** l'au moins un élément de piqûre est plié pour son utilisation.

12. Système selon la revendication 11, **caractérisé en ce que** l'endroit auquel l'élément de piqûre est plié est choisi avant l'utilisation.

13. Système selon l'une quelconque des revendications précédentes, **caractérisé en ce que** l'au moins un élément de piqûre présente une protection stérile.

14. Système selon l'une quelconque des revendications précédentes, **caractérisé en ce que** l'ouverture présente une pièce de pression pour l'appui d'une partie du corps sur l'ouverture.
